# EUROPEAN PATENT APPLICATION

(11) **EP 4 324 465 A1**
(43) Date of publication of application: **21.02.2024**
(21) Application number: 22190381.8
(22) Date of filing: 15.08.2022
(51) Int. Cl.: A61K 31/7016, A61P 31/00, A61P 31/02, A61P 31/04, A61K 47/00

(54) **COMPOUNDS FOR USE IN THE TREATMENT OF A MICROBIAL INFECTION IN THE UROGENITAL SYSTEM**

(71) Applicant: Gedea Biotech AB, 223 81 Lund (SE)
(72) Inventor: ELLERVIK, Ulf, 246 33 Löddeköpinge (SE); STREVENS, Helena, 227 36 Lund (SE)
(74) Representative: Høiberg P/S

(57) **Abstract**

The present invention relates to compounds for use in the treatment and/or prevention of microbial infections in the urogenital system.

## Description

### Technical field

The present invention relates to compounds for use in the treatment and/or prevention of microbial infections in the urogenital system.

### Background

The female reproductive tract environment and especially the vaginal microbiome is a dynamic system with a complex mixture of various microorganisms in different ratios and quantities. In the healthy state, the vaginal flora is predominantly colonized by *Lactobacilli spp.,* providing protection against pathological colonization, mainly through excretion of lactic acid, which maintains a weakly acidic environment (typically pH 3.5 to 4.5) and antimicrobial compound production (Reid et al, Nat Rev Microbiol 2011, 9, 1, 27-38). Any sudden change in the vaginal microflora will increase the vaginal pH, and consequently create a more favourable environment for the establishment of vaginal pathogens, which grow optimally at a pH over 5. The imbalance in the flora of microorganisms in the vagina can thus lead to dysbiosis and vaginal infections, caused by pathogenic bacteria or *Candida,* conditions that affect a large proportion of women of reproductive age each year and may negatively affect fertility and the ability to maintain a healthy pregnancy. A disrupted reproductive tract microbiome has been associated with unfavourable outcomes of pregnancy and reproductive complications. Ascending bacterial infection from the vagina through the cervix into the uterine cavity is considered to be a major cause of preterm birth (Goldenberg et al, Lancet, 2008, 37, 9606, 75-849; Maclntyre et al, Sci Rep, 2015, 5, 8988). Many of these cases also exhibit chorioamnionitis, constituting a major threat also to the mother, before the pathogen is identified and effective antibiotic treatment is commenced. The link between vaginal microbial community structure and ascending infection is also highlighted in late miscarriage, in recurrent spontaneous abortion, in chronic cervicitis/endometritis leading to implantation problems and female infertility. A healthy vaginal microbiome during pregnancy should be dominated by only one or a few *Lactobacillus* species (Maclntyre et al, Sci Rep, 2015, 5, 8988, Romero et al, Microbiome, 2014, 2, 1,4).

Current treatments of vaginal infections include vaginal capsules, tablets and creams such as econazol, clomitrazol, miconazole, tioconazole, butoconazol and fluconazole. However, many of the treatments currently on the market accompanied a high recurrence of the infection, and in addition have various side effects, including stomach upset, headache and rashes. Some of the treatments cannot be used during gestation due to the potential risk of harm to the fetus. Furthermore, a resistance to the drug may develop as the infectious organisms the antimicrobial agent is designed to kill adapt to them. In addition, many of the treatments are pharmaceutical fat based pessaries, which are not stable at elevated temperatures. Thus, there is a need for novel, treatment-and-prevention alternatives, which are safe during pregnancy and with low risk of resistance development.

### Summary

The present inventors have developed a method for treatment and/or prevention of microbial infections in the urogenital system. As demonstrated herein, vaginal treatment with a pharmaceutical tablet formulation of isomalt reduced the most prominent symptoms of bacterial vaginosis in over 70% of the patients in a clinical trial.

In one aspect, the present invention relates to a compound or a mixture of compounds selected from the group consisting of a monosaccharide, a sugar alcohol and a monosaccharide conjugated to a sugar alcohol for use in the treatment and/or prevention of a microbial infection in the urogenital system in a subject. In a preferred embodiment, the compound or mixture of compounds is isomalt or a pharmaceutically acceptable salt thereof.

In a further aspect, the present invention relates to a compound or a mixture of compounds selected from the group consisting of a monosaccharide, a sugar alcohol and a monosaccharide conjugated to a sugar alcohol for use in the prevention of late miscarriage or preterm birth. In a preferred embodiment, the compound or mixture of compounds is isomalt or a pharmaceutically acceptable salt thereof.

I another aspect, the present invention relates to a pharmaceutical composition comprising:
a. 10 to 50 wt% isomalt or a pharmaceutically acceptable salt thereof; and
b. 5 to 40 wt% Hypromellose (HPMC).

### Description of Drawings

**Figure 1****.** Growth of L.iners in the absence (empty circles) or presence (black squares) of 2.5% isomalt as measured by OD600. The experiment was performed with oil overlay, i.e. in anaerobic conditions.
**Figure 2****.** Growth of L.iners for 72h in the presence (black bars) or absence (white bar) of isomalt in different concentrations as measured by OD600.
**Figure 3****.** Growth of L.crispatus in the absence (empty circles) or presence (black squares) of 0.5% isomalt as measured by OD600. The experiment was performed without oil overlay, i.e. in aerobic conditions.
**Figure 4****.** Growth of L.crispatus in the absence (empty circles) or presence (black squares) of 2.5% lactitol as measured by OD600. The experiment was performed without oil overlay, i.e. in aerobic conditions.

### Detailed description

In one aspect, the present invention concerns a compound or a mixture of compounds selected from the group consisting of a monosaccharide, a sugar alcohol and a monosaccharide conjugated to a sugar alcohol for use in the treatment and/or prevention of a microbial infection in the urogenital system in a subject.

The term "urogenital system", also known as the urogenital tract or the genitourinary system, as used herein refers to the organs of the reproductive system and the urinary system.

A microbial infection in the urogenital system may be caused by an imbalance in the flora of microorganisms. In one embodiment, the microbial infection is treated or prevented by promoting the growth of one or more *Lactobacillus* species, which changes the vaginal environment to inhibit growth of pathogenic bacteria.

### Microbial infection

In one embodiment, the microbial infection is a fungal infection. In one embodiment, the microbial infection is a candida infection. In one embodiment, the candida infection is a vaginal candida infection.

In one embodiment, the microbial infection is a bacterial infection. In one embodiment, the microbial infection in the urogenital system is bacterial vaginosis.

### Infections of the reproductive system

The term "reproductive system", also known as the reproductive tract, the genital system or the genital tract, is the biological system made up of all the anatomical organs involved in sexual reproduction. The female genital system includes the uterus, ovaries, uterine tubes, vagina and clitoris.

In one embodiment, the infection in the urogenital system is an infection in the reproductive system. In one embodiment, the microbial infection in the urogenital system is a disease of the female genital system. In one embodiment, the infection in the urogenital system is vaginal infection.

In one embodiment, the disease of the female genital system is endometritis, such as chronic endometritis. Endometritis is an inflammation or irritation of the lining of the uterus (the endometrium).

Chronic endometritis is a persistent inflammation of the endometrial mucosa caused by bacterial pathogens such as Enterobacteriaceae, Enterococcus, Streptococcus, Staphylococcus, Mycoplasma, and Ureaplasma. Although chronic endometritis can be asymptomatic, it is found in up to 40% of infertile patients and is responsible for repeated implantation failure and recurrent miscarriage (Moreno, I. et al. (2018) Am J Obstet Gynecol;218:602.e1-16). Diagnosis and treatment of chronic endometritis improve spontaneous pregnancy rate and live birth rate in patients with unexplained infertility (Cicinelli, E. et al., (2018) Am J Reprod Immunol. 2018, 79(1)). Thus, by treating the microbial infection in these infertile patients, the infertility is also treated.

In one embodiment, the disease of the female genital system is female infertility. The term "female infertility" as used herein refers to a disease of the reproductive system defined by the failure to achieve a clinical pregnancy after 12 months or more of regular unprotected sexual intercourse. In one embodiment, the infertility is due to implantation failure, such as recurrent implantation failure. In one embodiment, the infertility is due to spontaneous abortions, such as recurrent spontaneous abortions. A spontaneous abortion is characterised by non-induced embryonic or fetal death or passage of products of conception prior to 22 weeks gestation or weighing less than 500 grams.

In one aspect, the present invention concerns a compound selected from the group consisting of a monosaccharide, a sugar alcohol and a monosaccharide conjugated to a sugar alcohol for use in enhancing fertility in a subject.

In one embodiment, the disease of the female genital system is cervicitis, such as chronic cervicitis. Cervicitis is an inflammation of the cervix, the lower, narrow end of the uterus that opens into the vagina.

### Prevention of late miscarriage and preterm birth

Infection has long been recognised as an important risk factor of poor reproductive success. There is evidence that up to 15% of early miscarriages and up to 66% of late miscarriages are caused by infections (Giakoumelou, S. et al, (2016) Hum Reprod Update, 22(1):116-133). Towards the end of pregnancy, infection is associated with approximately 40% of all preterm birth cases (Grewal, K. (2021) Biosci Rep., 41(9)).

In one embodiment, the microbial infection is a vaginal bacterial vulvovaginitis. With cervical ripening or cervical insufficiency, the said infection may migrate to the uterus and cause chorioamnionitis and subsequently preterm birth. There is evidence supporting that excessive inflammation, such as a vaginitis or cervicitis, through prostaglandin production can cause premature contractions and preterm birth even in the absence of manifest chorioamnionitis. The preterm neonate can subsequently face invasive bacterial infection; pneumonia, meningitis or sepsis, due to neonatal immunodeficiency in the preterm infant. Especially group A and B streptococci and multiresistent bacteria can give rise to serious perinatal infection in the infant and postpartum endometritis in the newly delivered woman and be the cause of both neonatal and maternal serious morbidity and mortality.

In another embodiment, the microbial infection is vulvovaginal candidiasis. The preterm neonate may face invasive *Candida* infection, one of the most serious nosocomial infections causing higher morbidity and mortality than bacterial infection, in particular in neonatal intensive care units.

In one aspect, the present invention concerns a compound selected from the group consisting of a monosaccharide, a sugar alcohol and a monosaccharide conjugated to a sugar alcohol for use in the prevention of late miscarriage. A late miscarriage is one that happens after the first 12 weeks of pregnancy, but before 24 weeks. In one embodiment, the miscarriage is caused by a microbial infection.

In one aspect, the present invention concerns a compound selected from the group consisting of a monosaccharide, a sugar alcohol and a monosaccharide conjugated to a sugar alcohol for use in the prevention of preterm birth. In one embodiment, the preterm birth is caused by a microbial infection. In one embodiment, the preterm birth is extremely preterm, very preterm or moderate preterm, such as early moderate preterm or late moderate preterm.

A preterm birth is classified as "extremely preterm" when the gestation lasts less than 28 weeks. A preterm birth is classified as "very preterm" when the gestation lasts 28 to under 32 weeks. A preterm birth is classified as "moderate preterm" when the gestation lasts 32 to under 37 weeks. A preterm birth is classified as "early moderate preterm" when the gestation lasts 32 to under 34 weeks. A preterm birth is classified as "late moderate preterm" when the gestation lasts 34 to under 37 weeks.

### Reduction of abnormal and/or odorous vaginal discharge and/or vaginal discomfort

In one aspect, the present invention concerns a compound selected from the group consisting of a monosaccharide, a sugar alcohol and a monosaccharide conjugated to a sugar alcohol for use in reducing abnormal and/or odorous vaginal discharge and/or vaginal discomfort in a subject.

### Infections of the urinary system

The term "urinary system", also known as the urinary tract or renal system, consists of the kidneys, ureters, bladder, and the urethra.

In one embodiment, the infection in the urogenital system is an infection of the urinary system. In one embodiment, the microbial infection in the urogenital system is selected from the group consisting of urinary tract infection, urinary urge incontinence and cystitis, such as chronic cystitis. Cystitis is a condition of the bladder, caused by infection, reaction to pharmacological agents, exposure to radiation therapy, or potential irritants. This condition is characterised by inflammation of the urinary bladder, dysuria, pollakiuria, fever, or flank pain.

### The subject

In one embodiment, the subject is a mammal. In one embodiment, the mammal is a human. In one embodiment, the human is a female. In one embodiment, the female is pregnant. In one embodiment, the subject is suffering from a microbial infection in the urogenital system.

### The compound

The present invention relates to use of a compound or a mixture of compounds selected from the group consisting of a monosaccharide, a sugar alcohol and a monosaccharide conjugated to a sugar alcohol.

The term "sugar alcohol" (also referred to as "hydrogenated sugar") as used herein refers to a compound obtained by adding hydrogen to the reductive end group in sugar, i.e. a sugar derivative whose carbonyl group (aldehyde or ketone, reducing sugar) has been reduced to a primary or secondary hydroxyl group. In general, a sugar alcohol has a chemical formula of HOCH₂(CHOH)ₙCH₂OH wherein n is an integer of 2 to 5. Examples of sugar alcohols of the present invention include but are not limited to glucitol, mannitol, xylitol and erythritol:

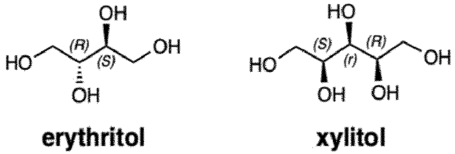

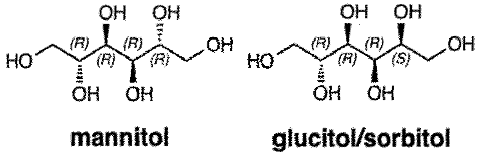

The term "saccharide" as used herein refers to a sugar, and the term "monosaccharide" refers to one sugar unit. Examples of monosaccharides of the present invention include but are not limited to glucose and galactose:

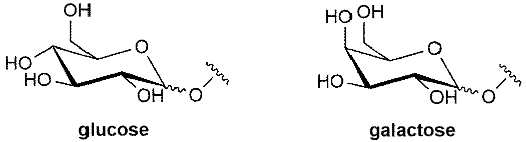

In one embodiment, the monosaccharide is the alpha anomer. In one embodiment, the monosaccharide is the beta anomer. In one embodiment, the monosaccharide is alpha-glucose. In one embodiment, the monosaccharide is beta-glucose.

In one embodiment, the compound is a monosaccharide conjugated to a sugar alcohol. Preferably, the monosaccharide is conjugated via its anomeric position to the sugar alcohol. In one embodiment, the sugar alcohol is conjugated via position 6 to the monosaccharide. In one embodiment, the sugar alcohol is conjugated via position 4 to the monosaccharide. In one embodiment, the sugar alcohol is conjugated via position 1 to the monosaccharide. In one embodiment, the monosaccharide is conjugated via its anomeric position to position 6, 4 or 1 of the sugar alcohol. In one embodiment, the monosaccharide is conjugated to the sugar alcohol via an alpha glycosidic bond. In one embodiment, the monosaccharide is conjugated to the sugar alcohol via a beta glycosidic bond.

In one embodiment, the monosaccharide is glucose and the sugar alcohol is glucitol or mannitol.

In one embodiment, the compound is selected from the group consisting of:

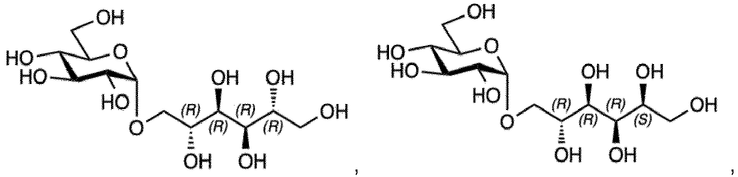

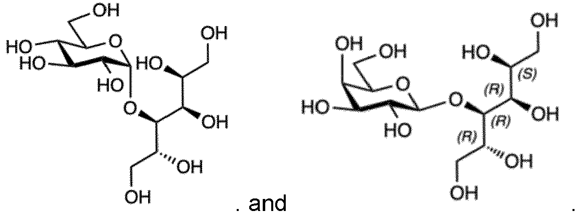

In one embodiment, the monosaccharide is galactose and the sugar alcohol is glucitol.

In one embodiment, the compound is glucitol optionally conjugated to glucose or galactose.

In one embodiment, the compound is mannitol optionally conjugated to glucose.

In one embodiment, the compound is selected from the group consisting of isomalt, maltitol, lactitol, glucitol, mannitol, xylitol, erythritol and lactulose; or a pharmaceutically acceptable salt thereof.

In one embodiment, the compound or mixture of compounds is isomalt. Isomalt is an equimolar mixture of two diastereomeric disaccharides, each composed of two sugars: glucose and mannitol (α-D-glucopyranosido-1,6-mannitol) and also glucose and sorbitol (α-D-glucopyranosido-1,6-sorbitol):

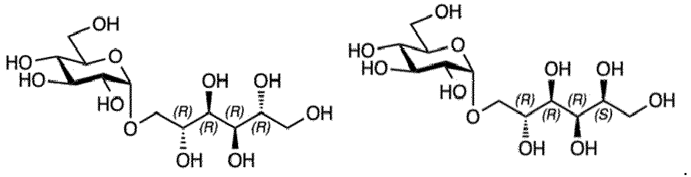

In one embodiment, the compound is α-D-glucopyranosido-1,6-mannitol. In one embodiment, the compound is α-D-glucopyranosido-1,6-sorbitol.

In one embodiment, the compound is maltitol (4-*O*-α-glucopyranosyl-D-sorbitol):

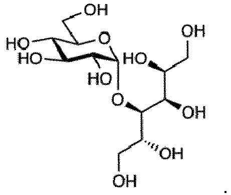

In one embodiment, the compound is lactitol (4-O-α-D-Galactopyranosyl-D-glucitol):

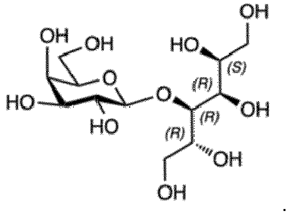

### Pharmaceutical composition

In one aspect, the present invention concerns a pharmaceutical composition comprising the compound or mixture of compounds selected from the group consisting of a monosaccharide, a sugar alcohol and a monosaccharide conjugated to a sugar alcohol.

As used herein, the term "pharmaceutical composition" refers to a composition capable of inducing a desired therapeutic effect in a subject. In certain embodiments, a pharmaceutical composition includes an active agent, which is the agent that induces the desired therapeutic effect. In certain embodiments, a pharmaceutical composition includes inactive ingredients such as carriers and excipients.

In one embodiment, the compound or mixture of compounds selected from the group consisting of a monosaccharide, a sugar alcohol and a monosaccharide conjugated to a sugar alcohol is in a composition, such as a pharmaceutical composition.

In one embodiment, the pharmaceutical composition comprises at least 5 wt %, such as at least 10 wt %, such as at least 15 wt %, such as at least 20 wt %, such as at least 25 wt %, such as at least 30 wt %, such as at least 40 wt %, such as at least 50 wt %, such as at least 60 wt % of the compound or mixture of compounds. In one embodiment, the pharmaceutical composition comprises no more than 99 wt %, such as no more than 95 wt %, such as no more than 90 wt %, such as no more than 85 wt %, such as no more than 80 wt %, such as no more than 75 wt % of the compound or mixture of compounds. In one embodiment, the pharmaceutical composition comprises 5 to 99 wt %, such as in 10 to 95 wt %, such as in 15 to 95 wt %, such as in 20 to 90 wt %, such as in 40 to 95 wt %, such as in 40 to 95 wt %, such as in 50 to 95 wt % of the compound. In one embodiment, the composition comprises 15 to 45 wt% of the compound, such as about 20 wt% to 40 wt%, such as 30 to 35 wt% or such as about 33 wt% of the compound or mixture of compounds.

In one embodiment, the amount of compound or mixture of compounds in the pharmaceutical composition is 50 to 1500 mg, such as 50 to 1000 mg, such as 50 to 500 mg, such as 100 to 400 mg, such as 250 to 350 mg, such as about 330 mg.

In one embodiment, the pharmaceutical composition comprises no more than 10 wt % water, such as no more than 5 wt % water.

In one embodiment, no more than 60 % of the pharmaceutical composition is dissolved within two hours using United States Pharmacopeia (USP) Dissolution Apparatus 2 - Paddle method, at 50 rpm in 500 mL 50 mM acetate buffer (pH 4.0) at 37°C. In one embodiment, the compound or mixture of compounds is released *in vivo* for at least 6 h, such as for at least 12 h, such as for at least 18 h, such as for at least 24 h, such as for at least 36 h, such as for at least 42 h.

In one embodiment, the pharmaceutical composition is adapted for administration for at least once daily during at least a week. In one embodiment, the pharmaceutical composition is formulated to release the compound over an extended period of time, such as over at least 4 hours, such as over at least 6 hours, such as over at least 24 hours after administration. In one embodiment, the pharmaceutical composition is adapted for administration at least once daily, such as at least twice daily, such as at least three times daily. In one embodiment, the pharmaceutical composition is adapted for administration no more than every second day, such as no more than every third day, such as no more than once a week. In one embodiment, the pharmaceutical composition is adapted for administration for no more than six days. In one embodiment, the pharmaceutical composition is adapted for administration during at least one week, such as during at least two weeks, such as during at least three weeks, such as during at least four weeks.

In one embodiment, the pharmaceutical composition is formulated as a tablet, orally disintegrating tablet, lozenge, gum, chewing gum, cream, lotion, gel, emulsion, solution, foam, ointment, spray, rinse, suspension mouthwash, nail polish, dermal patch or shampoo. In one embodiment, the pharmaceutical composition is a solid pharmaceutical composition. In one embodiment, the pharmaceutical composition is a tablet.

In one embodiment, the pharmaceutical composition is suitable for vaginal administration. In one embodiment, the pharmaceutical composition is formulated as a tampon, vagitorium, vaginal aerosol, vaginal cup, vaginal gel, vaginal insert, vaginal patch, vaginal ring, vaginal sponge, vaginal suppository, vaginal cream, vaginal emulsion, vaginal foam, vaginal lotion, vaginal ointment, vaginal powder, vaginal shampoo, vaginal solution, vaginal spray, vaginal suspension, vaginal tablet, vaginal rod, vaginal disc, vaginal device, uterine flushing, uterine wash, uterine oil, uterine rinse and any combination thereof. In one embodiment, the composition is present on a sanitary article, such as a tampon, a sanitary napkin, an incontinence pad or diaper, or a panty liner.

In one embodiment, the compound or mixture of compounds is the sole active pharmaceutical ingredient in the composition. In one embodiment, the composition comprises no further active pharmaceutical ingredient, such as no antiviral active pharmaceutical agent, no antibacterial active pharmaceutical ingredient or no antifungal active pharmaceutical ingredient.

In one embodiment, the pharmaceutical composition comprises or consists of isomalt, or a pharmaceutically acceptable salt thereof, and one or more non-antimicrobial agents. In one embodiment, the non-antimicrobial agent is an agent for formulation as a tampon, vagitorium, vaginal aerosol, vaginal cup, vaginal gel, vaginal insert, vaginal patch, vaginal ring, vaginal sponge, vaginal suppository, vaginal cream, vaginal emulsion, vaginal foam, vaginal lotion, vaginal ointment, vaginal powder, vaginal shampoo, vaginal solution, vaginal spray, vaginal suspension, vaginal tablet, vaginal rod, vaginal disc, vaginal device, and any combination thereof, or as a sanitary article, such as a tampon, a sanitary napkin, an incontinence pad or diaper, or a panty liner. In one embodiment, the non-antimicrobial agent is a pharmaceutically acceptable carrier, excipient, diluent and/or adjuvant known and used in the art.

### Hypromellose (HPMC)

In one embodiment, the pharmaceutical composition comprises at least one hypromellose (HPMC) component. The HPMC component act as a release-modifying agent in the pharmaceutical composition. The term "hypromellose" as used herein refers to hydroxypropyl methylcellulose, CAS number 9004-65-3, E number E464. HPMC is a partly O-methylated and O-(2-hydroxypropylated) cellulose and is available in several grades that differ in molecular weight as well as in the extent of substitution, and therefore also differ in viscosity. HPMC types may be classified based on the extent of substitution, and thus given a four digit number. The first two digits refer to the percentage (w/w) of the methoxy, while the second two digits refer to the percentage of the hydroxypropoxy-groups in the dried substance. In one embodiment, the HPMC component of the present invention is selected from the group consisting of HPMC substitution types 2208 (also known as "K"), 2910 (also known as "E"), 1828 and 2906 (also known as "F"). In one embodiment, the HPMC component is of substitution type 2208 or 2910.

The structure of the HPMC, including the size and extent of substitution, gives rise to viscoelastic properties. In addition to the substitution pattern, the different HPMC grades can be distinguished by the apparent viscosity (mPas) of a 2% (w/w) aqueous solution. In one embodiment, the HPMC component has a viscosity of at least 10 mPas, such as at least 15 mPas, such as at least 20 mPas, such as at least 25 mPas, such as at least 30 mPas, such as at least 35 mPas, such as at least 40 mPas, such as at least 45 mPas, such as at least 50 mPas, such as at least 55 mPas, such as at least 60 mPas, such as at least 65 mPas, such as at least 70 mPas, such as at least 75 mPas. In one embodiment, the HPMC component has a viscosity of at least 50 mPas. In one embodiment, the HPMC component has a viscosity of no more than 400.000 mPas, such as no more than 300.000 mPas, such as no more than 200.000 mPas, such as no more than 150.000 mPas, such as no more than 100.000 mPas, such as no more than 50.000 mPas, such as no more than 10.000 mPas, such as no more than 1.000 mPas, such as no more than 500 mPas, such as no more than 250 mPas. In one embodiment, the HPMC component has a viscosity in the range of 80 to 280.000 mPas, such as in the range of 80 to 120mPas, such as in the range of 2.600 to 5.000 mPas, or such as in the range of 150.000 to 280.000 mPas. In one embodiment, the HPMC component has a viscosity of about 100.000 mPas. In one embodiment, the HPMC component has a viscosity of about 200.000 mPas. In one embodiment, the HPMC component has a viscosity of about 4000 mPas. In one embodiment, the HPMC component has a viscosity of about 100 mPas. In one embodiment, the HPMC component has a viscosity of about 15 mPas. In one embodiment, the HPMC viscosity is in the range of 50 to 200 mPas, such as 100 mPas. In one embodiment, the HPMC viscosity is in the range of 50.000 to 200.000 mPas, such as 100.000 mPas.

In one embodiment, the HPMC component may be selected from the group consisting of Methocel K100, Methocel K4M and Methocel K200M.

In one embodiment, the pharmaceutical composition comprises at least 5 wt% HPMC, such as at least 10 wt%, such as at least 10 wt%, such as at least 15 wt%, such as at least 20 wt%, such as at least 25 wt%, such as at least 30 wt%, such as at least 35 wt%, such as at least 40 wt%, such as at least 45 wt%, such as at least 50 wt% HPMC. In one embodiment, the pharmaceutical comprises no more than 60 wt% HPMC, such as no more than 55 wt%, such as no more than 50 wt%, such as no more than 45 wt%, such as no more than 45 wt%, such as no more than 40 wt%, such as no more than 35 wt%, such as no more than 30 wt%, such as no more than 25 wt%, such as no more than 20 wt%, such as no more than 15 wt%, such as no more than 10 wt% HPMC. In one embodiment, the pharmaceutical composition comprises in the range of 5 to 60 wt% HPMC, such as 15 to 40 wt%, such as 5 to 25 wt%, such as 10 to 20 wt%, such as 10 to 40 wt%, such as 20 to 35 wt%, such as 30 to 40 wt% HPMC. In one embodiment, the pharmaceutical composition comprises in the range of 5 to 30 wt% HPMC, such as 10 to 20 wt%, such as about 15 wt% HPMC. In one embodiment, the pharmaceutical composition comprises in the range of 25 to 45 wt% HPMC, such as 30 to 30 wt%, such as about 35.5 wt%. In one embodiment, the composition comprises in the range of 10 to 20 wt% HPMC, such as about 15 wt% HPMC.

### Silica

In some embodiments, the pharmaceutical composition further comprises a glidant, such as silica. In some embodiments, the pharmaceutical composition further comprises silica. Said silica may be selected from the group consisting of colloidal anhydrous silica, colloidal silicone dioxide, colloidal hydrated silica and hydrophobic colloidal silica. In one embodiment, said silica is colloidal silica. In one embodiment, said silica has a specific surface area of 175 to 225 m²/g, such as about 200 m²/g, and/or an average primary particle size of 12 nm. In one embodiment, the silica has a pH value of 3.7 to 4.5 in 4% dispersion. In one embodiment, said silica is Aerosil 200.

In one embodiment, the pharmaceutical composition comprises 0.1 to 5 wt% silica, such as 0.1 to 2 wt%, such as 0.3 to 0.6 wt%, such as about 0.5 wt%, such as about 0.38 wt% silica.

### Lubricant

In some embodiments, the pharmaceutical composition further comprises a lubricant. Said lubricant may be selected from the group consisting of magnesium stearate, calcium stearate, stearic acid, sodium stearylfumarate, glyceryl behenate, glyceryl monooloeate, glyceryl monostearate, glyceryl palmitostearate, sucrose palmitate and sucrose stearate. In one embodiment, said lubricant is magnesium stearate.

In one embodiment, the pharmaceutical composition comprises 0.1 to 5 wt% lubricant, such as 0.1 to 2 wt%, such as 0.70 to 1.3 wt%, such as about 1 wt%, such as about 0.75 wt%, such as about 0.38 wt% lubricant.

### Starch

In some embodiments, the pharmaceutical composition further comprises one or more fillers, such as starch. In some embodiments, the pharmaceutical composition further comprises starch. In one embodiment, said starch is pregelatinized starch. In one embodiment, said starch is a partially pregelatinized maize starch. In one embodiment, said starch comprises amylopectin and amylose, such as in a 3:1 ratio. The gelatinization level of said starch may be about 20 %. The particle size of said starch may be about 65 micron. In one embodiment, said starch is Starch 1500^{®}.

In one embodiment, the pharmaceutical composition comprises at least 10 wt% starch, such as at least 15 wt%, such as at least 20 wt%, such as at least 30 wt %, such as at least 40 wt%. In one embodiment, the pharmaceutical composition comprises no more than 45 wt% starch, such as no more than 40 wt%, such as no more than 35 wt%, such as no more than 30 wt%, such as no more than 25 wt%, such as no more than 20 wt%. In one embodiment, the pharmaceutical composition comprises 10 to 50 wt% starch, such as 10 to 30 wt%, such as 10 to 25 wt%, such as 10 to 20 wt%, such as about 19 wt%, such as about 14.25 wt% starch.

### Cellulose

In some embodiments, the pharmaceutical composition further comprises one or more fillers, such as cellulose In some embodiments, the pharmaceutical composition further comprises cellulose. Said cellulose may be selected from the group consisting of microcrystalline cellulose (MCC), silicified microcrystalline cellulose and powder cellulose. In one embodiment, said cellulose is MCC. In one embodiment, said MCC has a nominal particle size of at least 20 µm, such as at least 50 µm, such as at least 75 µm, such as at least 100 µm, such as at least 125 µm, such as at least 140 µm, such as at least 150 µm, such as at least 160 µm. In one embodiment, said MCC has a nominal particle size of no more than 200 µm, such as no more than 190 µm, such as no more than 180 µm, such as no more than 170 µm, such as no more than 160 µm. In one embodiment, said MCC has a nominal particle size in the range of 140 to 200 µm, such as about 150 to about 180 µm.

In one embodiment, the pharmaceutical composition comprises at least 20 wt% cellulose, such as at least 30 wt%, such as at least 40 wt%, such as at least 50 wt% cellulose. In one embodiment, the pharmaceutical composition comprises no more than 60 wt% cellulose, such as no more than 50 wt%, such as no more than 40 wt%, such as no more than 30 wt% cellulose. In oneembodiment, the pharmaceutical comprises 20 to 60 wt% cellulose, such as 20 to 30 wt%, such as 40 to 60 wt%, such as50 to 60 wt%, such as 30 to 40 wt%, such as 35 to 40 wt%, such as about 36 wt% cellulose.

### Amounts

The amount of the various components of the pharmaceutical composition is sometimes given in the present disclosure in wt%. In such cases, the sum of the wt% of the components does not exceed 100 wt%. In some embodiments, the pharmaceutical composition comprises or consists of the compound, HPMC, starch, silica and magnesium stearate.

In one embodiment, the pharmaceutical composition comprises:
a. 10 to 50 wt% of the compound or mixture of compounds; and
b. 5 to 40 wt% Hypromellose (HPMC).

In one embodiment, the composition comprises or consists of: 10 to 50 wt% of the compound or mixture of compounds; 10 to 40 wt% HPMC; 10 to 25 wt% starch; 0.25 to 1.25 wt% silica; and 0.5 to 1.5 wt% magnesium stearate, with the proviso that the sum of the components does not exceed 100 wt%.

In one embodiment, the composition comprises or consists of: 10 to 50 wt% of the compound or mixture of compounds; 5 to 40 wt% HPMC; 10 to 45 wt% MCC; 10 to 20 wt% starch; 0.25 to 1.25 wt% silica; and 0.5 to 1.5 wt% magnesium stearate.

In one embodiment, the pharmaceutical composition comprises or consists essentially of about: 30 to 35 wt% isomalt; 10 to 20 wt% HPMC; 30 to 40 wt% MCC; 12 to 18 wt% starch; 0.25 to 1.25 wt% silica; and 0.5 to 1.5 wt% magnesium stearate, with the proviso that the sum of the components does not exceed 100 wt%.

In one embodiment, the composition comprises or consists essentially of about: 33 wt% of the compound or mixture of compounds; 15 wt% HPMC; 36 wt% MCC; 14.25 wt% starch; 0.38 wt% silica; and 1.0 wt% magnesium stearate, with the proviso that the sum of the components does not exceed 100 wt%.

### Physical properties of the dosage form

In one embodiment, the pharmaceutical composition is in the form of a dosage form, such as a solid dosage form, for example a tablet. In one embodiment, the mass of the dosage form is at least 0.5 g, such as at least 0.7 g, such as at least 0.85 g, such as at least 1.0 g, such as at least 1.25 g, such as at least 1.4 g. In one embodiment, the mass of the dosage form is no more than 2.0 g, such as no more than 1.75 g, such as no more than 1.5 g, such as no more than 1.0 g, such as no more than 0.9 g. In one embodiment, the mass of the dosage form is in the range of 0.5 to 2.0 g, such as in the range of 0.7 to 1.75 g, such as in the range of 1.4 to 1.6 g. In one embodiment, the mass of the dosage form is about 1.5 g. In one embodiment, the mass of the dosage form is in the range of 0.5 to 2.0 g, such as in the range of 0.5 to 1.25 g, such as in the range of 0.7 to 1.0 g. In one embodiment, the mass of the dosage form is about 0.85 g. In one embodiment, the mass of the dosage form is about 1.0 g.

In one embodiment, the dosage form is bullet shaped.

In one embodiment, the dosage form has a width of at least 5 mm, such as at least 7 mm, such as at least 9 mm, such as at least 10 mm. In one embodiment, the dosage form has a width of no more than 15 mm, such as no more than 12 mm, such as no more than 11 mm, such as no more than 10 mm. In one embodiment, the dosage form has a width in the range of 8 to 12 mm, such as in the range of 8.5 to 11 mm, such as 10 to 10.5 mm. In one embodiment, the dosage form has a width in the range of 7.0 to 12 mm, such as in the range of 8.0 to 10 mm, such as 8.5 to 9.5 mm. In one embodiment, the dosage form has a width in the range of 10 to 10.5 mm or 8.5 to 9.5 mm.

In one embodiment, the dosage form has a length of at least 10 mm, such as at least 15 mm, such as at least 20 mm. In one embodiment, the dosage form has a length of no more than 30 mm, such as no more than 25 mm, such as no more than 20 mm. In one embodiment, the dosage form has a length in the range of 10 to 30 mm, such as in the range of 15 to 20 mm, such as in the range of 16 to 18 mm. In one embodiment, the dosage form has a length in the range of 10 to 30 mm, such as in the range of 22 to 27 mm, such as in the range of 24 to 25 mm. In one embodiment, the dosage form has a length in the range of 16 to 18 mm or 24 to 25 mm.

In one embodiment, the dosage form has a width in the range of 10 to 10.5 mm and a length of 24 to 25 mm. In another embodiment, the dosage form has a width in the range of 8.5 to 9.5 mm and a length of 16 to 18 mm. In another embodiment, the dosage form has a width in the range of 10.0 to 10.5 mm and a length of 16 to 18 mm.

In one embodiment, the amount of the compound or mixture of compounds in the dosage form is 50 to 500 mg, such as 100 to 400 mg, such as 250 to 350 mg, such as about 300 mg or about 150 mg.

In one embodiment, the breaking force for the pharmaceutical composition is in the range of 5 to 25 kp, such as 5 to 15 kp or 15 to 25 kp.

In one embodiment, the friability of the pharmaceutical composition is in the range of 0.01 to 1 %, such as 0.01 to 0.5 %, such as 0.1 to 0.2 % or such as 0.2 to 0.5 %.

In one embodiment, the pharmaceutical composition is stable for at least 6 months in 25 °C, such as for at least 6 months in 40 °C.

### Method for manufacturing

In one aspect, the present invention relates to a method for manufacturing the pharmaceutical composition as defined herein, said method comprises the steps:
a. Sieving the compound or mixture of compound as defined herein and HPMC;
b. Mixing the sieved components in a.; and
c. Compressing the powder mixture in b. to tablets.

In one embodiment, said method further comprises steps of mixing sieved silica with the sieved components of step a., and adding a lubricant, which is pre-mixed with a portion of the mixture generated in step b., i.e. the method comprises the steps:
a. Sieving the compound or mixture of compound as defined herein and HPMC;
b. Mixing sieved silica with the sieved components in a.;
c. Mixing lubricant with a portion of the mixture in b.;
d. Adding the mixture in c. to the remaining mixture in b. and mixing; and
e. Compressing the powder mixture in formed d. to tablets.

### Combination treatments

In one embodiment, the compound or mixture of compounds is administered in combination with one or more further agents, such as antimicrobial agents. In one embodiment, said antimicrobial agent is an antifungal agent or an antibacterial agent.

An "antimicrobial agent", as used herein, refers to an agent that is capable of decreasing or eliminating or inhibiting the growth of microorganisms such as that term is known in the art (exemplary microorganisms include microbes such as bacteria, fungi, viruses and other pathogens). Similarly, the term "antifungal agent" refers to an agent that is capable of decreasing or eliminating or inhibiting the growth of fungi, and the term "antibacterial agent" refers to an agent that is capable of decreasing or eliminating or inhibiting the growth of bacteria.

In one embodiment, the compound or mixture of compounds is administered in combination with one or more antibacterial agents. Antibiotics are a type of antimicrobial agents used in the treatment and prevention of bacterial infections. In one embodiment, the antibacterial agent is selected from the group consisting of clindamycin, tetracycline, amoxicillin, ampicillin, erythromycin, doxycycline, lumefloxacin, norfloxacin, afloxam, ciproflaxin, azitromycin, and cefltoxine.

In one embodiment, the compound or mixture of compounds is administered in combination with one or more antifungal agents. In one embodiment, the antifungal agent is selected form the group consisting of miconazole, terconazole, isoconazole, fenticonazole, fluconazole, nystatin, ketoconazole, clotrimazole, butoconazole, econazole, tioconazole, itraconazole, 5-fluoracil, and metronidazole.

In one embodiment, the compound or mixture of compounds is administered in combination with glucono-delta-lactone.

In one embodiment, the compound or mixture of compounds is administered in combination with a steroid, such as cortisone.

In one embodiment, the compound or mixture of compounds and the further agent are administered simultaneously, sequentially or separately. Sequential administration may be close in time or remote in time.

### Methods and uses

In one aspect, the present invention relates to a method for treating and/or preventing a microbial infection in the urogenital system, comprising administering a compound or a mixture of compounds selected from the group consisting of a monosaccharide, a sugar alcohol and a monosaccharide conjugated to a sugar alcohol to a subject in need thereof.

In one aspect, the present invention relates to a method for prevention of late miscarriage or preterm birth, comprising administering a compound or mixture of compounds selected from the group consisting of a monosaccharide, a sugar alcohol and a monosaccharide conjugated to a sugar alcohol to a subject in need thereof.

In one aspect, the present invention relates to a method for enhancing fertility, comprising administering a compound or mixture of compounds selected from the group consisting of a monosaccharide, a sugar alcohol and a monosaccharide conjugated to a sugar alcohol to a subject in need thereof.

In one aspect, the present invention relates to a method for reducing abnormal and/or odorous vaginal discharge and/or vaginal discomfort, comprising administering a compound or mixture of compounds selected from the group consisting of a monosaccharide, a sugar alcohol and a monosaccharide conjugated to a sugar alcohol to a subject in need thereof.

In one aspect, the present invention relates to use of a compound or mixture of compounds selected from the group consisting of a monosaccharide, a sugar alcohol and a monosaccharide conjugated to a sugar alcohol for the manufacture of a medicament for treatment and/or prevention of a microbial infection in the urogenital system in a subject.

In one aspect, the present invention relates to use of a compound or mixture of compounds selected from the group consisting of a monosaccharide, a sugar alcohol and a monosaccharide conjugated to a sugar alcohol for the manufacture of a medicament for prevention of late miscarriage or preterm birth.

In one aspect, the present invention relates to use of a compound or mixture of compounds selected from the group consisting of a monosaccharide, a sugar alcohol and a monosaccharide conjugated to a sugar alcohol for the manufacture of a medicament for enhancing fertility in a subject.

In one aspect, the present invention relates to use of a compound or mixture of compounds selected from the group consisting of a monosaccharide, a sugar alcohol and a monosaccharide conjugated to a sugar alcohol for the manufacture of a medicament for reducing abnormal and/or odorous vaginal discharge and/or vaginal discomfort in a subject.

As shown by bacterial growth data in Example 3, isomalt promotes the growth of L. iners and L.crispatus. In one aspect, the present invention relates to a method for promoting growth of bacteria of the genus Lactobacillus, comprising administering a compound or mixture of compounds selected from the group consisting of a monosaccharide, a sugar alcohol and a monosaccharide conjugated to a sugar alcohol to a subject in need thereof. In one embodiment, the Lactobacillus is selected from the group consisting of L. crispatus, L. iners, L. vaginalis, L. jenseneii, L.gasseri, L. fermentum, L. plantarum and L. mulieris.

### Items

1. A compound or mixture of compounds selected from the group consisting of a monosaccharide, a sugar alcohol and a monosaccharide conjugated to a sugar alcohol for use in the treatment and/or prevention of a microbial infection in the urogenital system in a subject.
2. The compound or mixture of compounds for use according to any one of the preceding items, wherein the microbial infection is a fungal infection.
3. The compound or mixture of compounds for use according to any one of the preceding items, wherein the microbial infection is a candida infection.
4. The compound or mixture of compounds for use according to item 3, wherein the candida infection is a vaginal candida infection.
5. The compound or mixture of compounds for use according to item 1, wherein the microbial infection is a bacterial infection.
6. The compound or mixture of compounds for use according to item 5, wherein the microbial infection in the urogenital system is bacterial vaginosis.
7. The compound or mixture of compounds for use according to any one of the preceding items, wherein the infection in the urogenital system is an infection in the reproductive system.
8. The compound or mixture of compounds for use according to any one of the preceding items, wherein the microbial infection in the urogenital system is a disease of the female genital system.
9. The compound or mixture of compounds for use according to any one of the preceding items, wherein the infection in the urogenital system is vaginal infection.
10. The compound or mixture of compounds for use according to item 8, wherein disease of the female genital system is female infertility.
11. The compound or mixture of compounds for use according to item 10, wherein the infertility is due to implantation failure, such as recurrent implantation failure.
12. The compound or mixture of compounds for use according to item 10, wherein the infertility is due to spontaneous abortions, such as recurrent spontaneous abortions.
13. The compound or mixture of compounds for use according to item 8, wherein the disease of the female genital system is cervicitis, such as chronic cervicitis.
14. The compound or mixture of compounds for use according to item 8, wherein the disease of the female genital system is endometritis, such as chronic endometritis.
15. The compound or mixture of compounds for use according to any one of items 1 to 5, wherein the infection in the urogenital system is an infection of the urinary system.
16. The compound or mixture of compounds for use according to item 15, wherein the microbial infection in the urinary system is selected from the group consisting of urinary tract infection, urinary urge incontinence and cystitis, such as chronic cystitis.
17. A compound or mixture of compounds selected from the group consisting of a monosaccharide, a sugar alcohol and a monosaccharide conjugated to a sugar alcohol for use in the prevention of late miscarriage or preterm birth.
18. The compound or mixture of compounds for use according to item 17, wherein the preterm birth is extremely preterm, very preterm or moderate preterm, such as early moderate preterm or late moderate preterm.
19. The compound or mixture of compounds for use according to any one of items 17 or 18, wherein the miscarriage or preterm birth is caused by a microbial infection.
20. A compound or mixture of compounds selected from the group consisting of a monosaccharide, a sugar alcohol and a monosaccharide conjugated to a sugar alcohol for use in enhancing fertility in a subject.
21. A compound or mixture of compounds selected from the group consisting of a monosaccharide, a sugar alcohol and a monosaccharide conjugated to a sugar alcohol for use in reducing abnormal and/or odorous vaginal discharge and/or vaginal discomfort in a subject.
22. The compound or mixture of compounds for use according to any one of the preceding items, wherein the subject is a mammal.
23. The compound or mixture of compounds for use according to item 22, wherein the mammal is a human.
24. The compound or mixture of compounds for use according to item 23, wherein the human is a female.
25. The compound or mixture of compounds for use according to item 24, wherein the female is pregnant.
26. The compound or mixture of compounds for use according to any one of the preceding items, wherein the compound is a monosaccharide conjugated to a sugar alcohol.
27. The compound or mixture of compounds for use according to item 26, wherein the monosaccharide is conjugated via its anomeric position to the sugar alcohol.
28. The compound or mixture of compounds for use according to any one of items 26 or 27, wherein the sugar alcohol is conjugated via position 6, 4 or 1 to the monosaccharide.
29. The compound or mixture of compounds for use according to any one of items 26 to 28, wherein the monosaccharide is conjugated via its anomeric position to position 6, 4 or 1 of the sugar alcohol.
30. The compound or mixture of compounds for use according to any one of items 26 to 29, wherein the monosaccharide is conjugated to the sugar alcohol via an alpha glycosidic bond.
31. The compound or mixture of compounds for use according to any one of items 26 to 30, wherein the monosaccharide is glucose and the sugar alcohol is glucitol or mannitol.
32. The compound or mixture of compounds for use according to any one of items 26 to 31, wherein the compound is selected from the group consisting of: 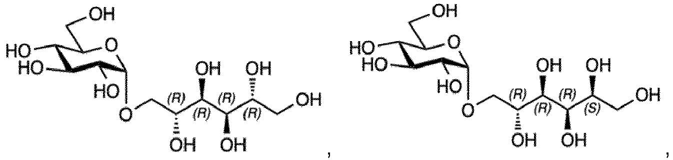 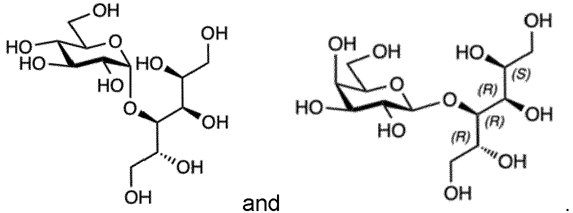
33. The compound or mixture of compounds for use according to any one of items 26 to 30, wherein the monosaccharide is galactose and the sugar alcohol is glucitol.
34. The compound or mixture of compounds for use according to any one of items 1 to 26, wherein the compound is glucitol optionally conjugated to glucose or galactose.
35. The compound or mixture of compounds for use according to any one of items 1 to 26, wherein the compound is mannitol optionally conjugated to glucose.
36. The compound or mixture of compounds for use according to any one of items 1 to 26, wherein the compound or mixture of compounds is selected from the group consisting of isomalt, maltitol, lactitol, glucitol, mannitol, xylitol, erythritol and lactulose; or a pharmaceutically acceptable salt thereof.
37. The compound or mixture of compounds for use according to any one of items 1 to 25, wherein the compound is isomalt or a pharmaceutically acceptable salt thereof.
38. The compound or mixture of compounds for use according to any one of the preceding items, wherein the compound or mixture of compounds is in a pharmaceutical composition.
39. The compound or mixture of compounds for use according to item 38, wherein the pharmaceutical composition is formulated as a tablet, orally disintegrating tablet, lozenge, gum, chewing gum, cream, lotion, gel, emulsion, solution, foam, ointment, spray, rinse, suspension mouthwash, nail polish, dermal patch or shampoo.
40. The compound or mixture of compounds for use according to item 38, wherein the pharmaceutical composition is formulated as a tampon, vagitorium, vaginal aerosol, vaginal cup, vaginal gel, vaginal insert, vaginal patch, vaginal ring, vaginal sponge, vaginal suppository, vaginal cream, vaginal emulsion, vaginal foam, vaginal lotion, vaginal ointment, vaginal powder, vaginal shampoo, vaginal solution, vaginal spray, vaginal suspension, vaginal tablet, vaginal rod, vaginal disc, vaginal device, uterine flushing, uterine wash, uterine oil, uterine rinse and any combination thereof.
41. The compound or mixture of compounds for use according to item 39, wherein the composition is present on a sanitary article, such as a tampon, a sanitary napkin, an incontinence pad or diaper, or a panty liner.
42. The compound or mixture of compounds for use according to any one of items 38 to 41, wherein the pharmaceutical composition comprises at least 5 wt %, such as at least 10 wt %, such as at least 15 wt %, such as at least 20 wt %, such as at least 25 wt %, such as at least 30 wt %, such as at least 40 wt %, such as at least 50 wt %, such as at least 60 wt % of the compound.
43. The compound or mixture of compounds for use according to any one of items 38 to 42, wherein the pharmaceutical composition comprises no more than 99 wt %, such as no more than 95 wt %, such as no more than 90 wt %, such as no more than 85 wt %, such as no more than 80 wt %, such as no more than 75 wt % of the compound.
44. The compound or mixture of compounds for use according to any one of items 38 to 43, wherein the pharmaceutical composition comprises 5 to 99 wt %, such as in 10 to 95 wt %, such as in 15 to 95 wt %, such as in 20 to 90 wt %, such as in 40 to 95 wt %, such as in 40 to 95 wt %, such as in 50 to 95 wt % of the compound.
45. The compound or mixture of compounds for use according to any one of items 38 to 44, wherein the pharmaceutical composition comprises no more than 10 wt % water, such as no more than 5 wt % water.
46. The compound or mixture of compounds for use according to any one of items 38 to 45, wherein, the pharmaceutical composition is adapted for administration for at least once daily during at least a week.
47. The compound or mixture of compounds for use according to any one of items 38 to 46 wherein the pharmaceutical composition is formulated to release the compound over an extended period of time, such as over at least 4 hours, such as over at least 6 hours, such as over at least 24 hours after administration.
48. The compound or mixture of compounds for use according to any one of items 38 to 47 wherein the pharmaceutical composition is adapted for administration at least once daily, such as at least twice daily, such as at least three times daily.
49. The compound or mixture of compounds for use according to any one of items 38 to 48, wherein the pharmaceutical composition is adapted for administration no more than every second day, such as no more than every third day, such as no more than once a week.
50. The compound or mixture of compounds for use according to any one of items 38 to 49, wherein the pharmaceutical composition is adapted for administration for no more than six days.
51. The compound or mixture of compounds for use according to any one of items 38 to 50, wherein the pharmaceutical composition is adapted for administration during at least one week, such as during at least two weeks, such as during at least three weeks, such as during at least four weeks.
52. The compound or mixture of compounds for use according to any one of items 38 to 51, wherein the pharmaceutical composition is a solid pharmaceutical composition.
53. The compound or mixture of compounds for use according to any one of items 38 to 52, wherein the pharmaceutical composition is suitable for vaginal administration.
54. The compound or mixture of compounds for use according to any one of items 38 to 53, wherein the pharmaceutical composition comprises:
   a. 10 to 50 wt% of the compound or mixture of compounds; and
   b. 5 to 40 wt% Hypromellose (HPMC).
55. The compound or mixture of compounds for use according to any one of items 38 to 54, wherein the pharmaceutical composition is a tablet.
56. The compound or mixture of compounds for use according to any one of items 38 to 55, wherein no more than 60 % of the pharmaceutical composition is dissolved within two hours using United States Pharmacopeia (USP) Dissolution Apparatus 2 - Paddle method, at 50 rpm in 500 mL 50 mM acetate buffer (pH 4.0) at 37°C.
57. The compound or mixture of compounds for use according to any one of items 38 to 56, wherein the compound is released *in vivo* for at least 6 h, such as for at least 12 h, such as for at least 18 h, such as for at least 24 h, such as for at least 36 h, such as for at least 42 h.
58. The compound or mixture of compounds for use according to any one of items 38 to 57, wherein the amount of compound is 50 to 1500 mg, such as 50 to 1000 mg, such as 50 to 500 mg, such as 100 to 400 mg, such as 250 to 350 mg, such as about 330 mg.
59. The compound or mixture of compounds for use according to any one of items 38 to 58, wherein the composition comprises 15 to 45 wt% of the compound, such as about 20 wt% to 40 wt%, such as 30 to 35 wt% or such as about 33 wt% of the compound.
60. The compound or mixture of compounds for use according to any one of items 38 to 59, wherein the composition comprises in the range of 10 to 20 wt% HPMC, such as about 15 wt% HPMC.
61. The compound or mixture of compounds for use according to any one of items 38 to 60, wherein the HPMC viscosity is in the range of 50 to 200 mPas, such as 100 mPas.
62. The compound or mixture of compounds for use according to any one of items 38 to 61, wherein the HPMC viscosity is in the range of 50.000 to 200.000 mPas, such as 100.000 mPas.
63. The compound or mixture of compounds for use according to any one of items 38 to 62, wherein the composition further comprises starch, silica and/or a lubricant.
64. The compound or mixture of compounds for use according to any one of items 38 to 63, wherein the composition further comprises cellulose, such as microcrystalline cellulose (MCC).
65. The compound or mixture of compounds for use according to item 38, wherein the composition comprises or consists of: 10 to 50 wt% of the compound; 10 to 40 wt% HPMC; 10 to 25 wt% starch; 0.25 to 1.25 wt% silica; and 0.5 to 1.5 wt% magnesium stearate, with the proviso that the sum of the components does not exceed 100 wt%.
66. The compound or mixture of compounds for use according to item 38, wherein the composition comprises or consists of: 10 to 50 wt% of the compound or mixture of compounds; 5 to 40 wt% HPMC; 10 to 45 wt% MCC; 10 to 20 wt% starch; 0.25 to 1.25 wt% silica; and 0.5 to 1.5 wt% magnesium stearate.
67. The compound or mixture of compounds for use according to item 38, wherein the pharmaceutical composition comprises or consists essentially of about: 30 to 35 wt% isomalt; 10 to 20 wt% HPMC; 30 to 40 wt% MCC; 12 to 18 wt% starch; 0.25 to 1.25 wt% silica; and 0.5 to 1.5 wt% magnesium stearate, with the proviso that the sum of the components does not exceed 100 wt%.
68. The compound or mixture of compounds for use according to item 38, wherein the composition comprises or consists essentially of about: 33 wt% of the compound; 15 wt% HPMC; 36 wt% MCC; 14.25 wt% starch; 0.38 wt% silica; and 1.0 wt% magnesium stearate, with the proviso that the sum of the components does not exceed 100 wt%.
69. The compound or mixture of compounds for use according to any one of items 38 to 68, wherein the compound is the sole active pharmaceutical ingredient in the composition.
70. The compound or mixture of compounds for use according to any one of items 38 to 68, wherein composition comprises no further active pharmaceutical ingredient, such as no antiviral active pharmaceutical agent, no antibacterial active pharmaceutical ingredient or no antifungal active pharmaceutical ingredient.
71. The compound or mixture of compounds for use according to any one of items 1 to 68, wherein the compound or mixture of compounds is administered in combination with one or more antifungal agents.
72. The compound or mixture of compounds for use according to item 71, wherein the antifungal agent is selected form the group consisting of miconazole, terconazole, isoconazole, fenticonazole, fluconazole, nystatin, ketoconazole, clotrimazole, butoconazole, econazole, tioconazole, itraconazole, 5-fluoracil, and metronidazole.
73. The compound or mixture of compounds for use according to any one of items 1 to 68 or 71 to 72, wherein the compound is administered in combination with one or more antibacterial agents.
74. The compound or mixture of compounds for use according to item 73, wherein the antibacterial agent is selected from the group consisting of clindamycin, tetracycline, amoxicillin, ampicillin, erythromycin, doxycycline, lumefloxacin, norfloxacin, afloxam, ciproflaxin, azitromycin, and cefltoxine.
75. The compound or mixture of compounds for use according to any one of items 1 to 68 or 71 to 74, wherein the compound is administered in combination with glucono-δ-lactone.
76. The compound or mixture of compounds for use according to any one of items 1 to 68 or 71 to 75, wherein the compound is administered in combination with a steroid.
77. The compound or mixture of compounds for use according to any one of items 1 to 68 or 71 to 76, wherein the compound is administered in combination with cortisone.
78. A method for treating and/or preventing a microbial infection in the urogenital system, comprising administering a compound or mixture of compounds selected from the group consisting of a monosaccharide, a sugar alcohol and a monosaccharide conjugated to a sugar alcohol to a subject in need thereof.
79. A method for prevention of late miscarriage or preterm birth, comprising administering a compound or mixture of compounds selected from the group consisting of a monosaccharide, a sugar alcohol and a monosaccharide conjugated to a sugar alcohol to a subject in need thereof.
80. A method for enhancing fertility, comprising administering a compound or mixture of compounds selected from the group consisting of a monosaccharide, a sugar alcohol and a monosaccharide conjugated to a sugar alcohol to a subject in need thereof.
81. A method for reducing abnormal and/or odorous vaginal discharge and/or vaginal discomfort, comprising administering a compound or mixture of compounds selected from the group consisting of a monosaccharide, a sugar alcohol and a monosaccharide conjugated to a sugar alcohol to a subject in need thereof.
82. Use of a compound or mixture of compounds selected from the group consisting of a monosaccharide, a sugar alcohol and a monosaccharide conjugated to a sugar alcohol for the manufacture of a medicament for treatment and/or prevention of a microbial infection in the urogenital system in a subject.
83. Use of a compound or mixture of compounds selected from the group consisting of a monosaccharide, a sugar alcohol and a monosaccharide conjugated to a sugar alcohol for the manufacture of a medicament for prevention of late miscarriage or preterm birth.
84. Use of a compound or mixture of compounds selected from the group consisting of a monosaccharide, a sugar alcohol and a monosaccharide conjugated to a sugar alcohol for the manufacture of a medicament for enhancing fertility in a subject.
85. Use of a compound or mixture of compounds selected from the group consisting of a monosaccharide, a sugar alcohol and a monosaccharide conjugated to a sugar alcohol for the manufacture of a medicament for reducing abnormal and/or odorous vaginal discharge and/or vaginal discomfort in a subject.
86. A method for promoting growth of bacteria of the genus Lactobacillus, comprising administering a compound or mixture of compounds selected from the group consisting of a monosaccharide, a sugar alcohol and a monosaccharide conjugated to a sugar alcohol to a subject in need thereof.
87. The method according to item 86, wherein the Lactobacillus is selected from the group consisting of L. crispatus, L. iners, L. vaginalis, L. jenseneii, L. gasseri, L. fermentum, L. plantarum and L mulieris.
88. A pharmaceutical composition comprising:
   a. 10 to 50 wt% of a compound or mixture of compounds selected from the group consisting of a monosaccharide, a sugar alcohol and a monosaccharide conjugated to a sugar alcohol; or a pharmaceutically acceptable salt thereof; and
   b. 5 to 40 wt% Hypromellose (HPMC).
89. The pharmaceutical composition according to item 88, wherein the pharmaceutical composition comprises or consists essentially of about: 33 wt% of the compound or mixture of compounds; 15 wt% HPMC; 36 wt% MCC; 14.25 wt% starch; 0.38 wt% silica; and 1.0 wt% magnesium stearate, with the proviso that the sum of the components does not exceed 100 wt%.
90. The pharmaceutical composition according to any one of items 88 or 89, wherein the mixture of compounds is isomalt.

### Examples

### Example 1 - Preparation of a pharmaceutical composition comprising isomalt

A pharmaceutical composition comprising isomalt and other components of table 1 was prepared.

**Table 1. Pharmaceutical composition components and amounts.**

| **Component** | **Amount / unit (mg)** | **Function** |
|---|---|---|
| Isomalt 721 | 334.0 | API |
| Hypromellose *Metolose 90SH-100SR* | 150.0 | Binder/Release-modifying agent |
| Starch, pregelatinised *Starch 1500* | 142.5 | Filler |
| Cellulose, microcrystalline *Avicel PH-200* | 360.0 | Filler |
| Silica colloidal anhydrous *Aerosil 200 Pharma* | 3.8 | Glidant |
| Magnesium stearate *Magnesiumstearat* | 10.0 | Lubricant |
| TOTAL: | 1000.3 | |

Mixing was performed in Turbula T2F 2 L glass vessel. First, isomalt, HPMC and starch were sieved through 1.00 mm screen prior to mixing. Afterwards, silica was premixed with a similar volume of HPMC by shaking in a plastic bag and sieving through 1.00 mm screen and added to the other four powders in the mixer vessel. Mixing was performed for 8 min at 32 rpm. Lubricant (MgSt) was premixed with a similar volume of the 8-min-powder-mixture and sieved through 1.00 mm, added to the rest of the 8-min-powder-mixture and mixed for 2 min at 23 rpm. The powder mixture was compressed to tablets in Korsch EK-0, a punch/die set, 10.1 x 17.9 mm, tablet weight approx.. 1.0 g.

The friability was tested in accordance with The European Pharmacopoeia 2.9.7. *Friability of uncoated tablets.* The friability (0.13-0.20 %) and breaking force (18.6-19.5 kp) of the obtained tablets were acceptable.

### Example 2 - Clinical performance in treating bacterial vaginosis

The clinical performance of the pharmaceutical formulation of Example 1 A in treating bacterial vaginosis (BV) in human patients were investigated in an open-label study. Participants were 30 adult non-pregnant women seen at gynaecological clinics in Sweden and UK, with confirmed BV according to fulfilment of at least three of the four Amsel's criteria, i.e.:
1. Thin, white, yellow, homogenous discharge
2. Clue cells on microscopy (more than 20 percent of epithelial cells)
3. pH of vaginal fluid above 4.5
4. Release of fishy odor, "i.e. a positive whiff test" when alkali (10% potassium hydroxide [KOH] solution) is added.

On Day 0, participants had a gynaecological examination, vaginal samples taken, and received the investigational product to be self-administered once daily on days 0-5. Participants were examined after 7 days with respect to BV signs and symptoms.

Cure was defined as absence of the three, non-pH related Amsel criteria, namely, discharge, clue cells and fishy odour. The absence of the fourth Amsel criterion; pH above 4.5, was not included in the definition of cure. Patients reported symptoms of bacterial vaginosis (fishy smell, burning sensation and vaginal irritation) according to a scoring scale (0-3) daily with a mobile phone application.

The results are presented in tables 2 and 3.

**Table 2. Clinical cure rate at Day 7.**

| | **Statistic** | **Isomalt-treatment Full Analysis set (FAS) (N=30)** | **Isomalt-treatment Per Protocol Set (PPAS) (N=24)** |
|---|---|---|---|
| Visit 2 Day 7 | Clinical Cure n (%) | 14 (46.7%) | 14 (58.3%) |
| | No clinical cure n (%) | 16 (53.3%) | 10 (41.7%) |

**Table 3. Proportion of patients with reduction in symptom score compared to day 0 (before treatment) and mean symptom scores in the full analysis set.**

| | **Fishy smell (%)** | **Burning sensation (%)** | **Irritation (%)** |
|---|---|---|---|
| Day 1 | 29,2 | 25 | 29,2 |
| Day 2 | 60,9 | 21,7 | 52,2 |
| Day 3 | 55 | 35 | 60 |
| Day 4 | 60,9 | 34,8 | 56,5 |
| Day 5 | 73,9 | 39,1 | 73,9 |
| Day 6 | 76,2 | 42,9 | 66,7 |
| Day 7 | 72,2 | 44,4 | 66,7 |
| Mean score day 0 | 2,1 | 0,6 | 1,5 |
| Mean score day 4 | 1,1 | 0,3 | 0,7 |
| Mean score day 7 | 0,8 | 0,1 | 0,5 |

### Conclusion

Vaginal treatment with a pharmaceutical tablet formulation of isomalt, administered once daily for 6 consecutive days, may thus treat and reduce symptoms of bacterial vaginosis, where the most prominent symptom, fishy smell, was reduced in over 70% of patients.

### Example 3 - Promoting growth of L.iners and L.crispatus

The vaginal bacterial strain L.iners and L.crispatus were cultured in glucose free broth and inoculated to a 96-well flat bottom plate. Different concentrations of isomalt or lactitol were added to appropriate wells. Medium was added to blank wells. Each experiment was performed in triplicate. Cultures with L.iners were covered with a layer of mineral oil to create an anaerobic environment. Plates were sealed and incubated on a shaker at 37 °C. The OD600 was measured at 6 or 12 hour intervals for 72 hours on a Spectromax ix3 after resuspending the cultures for homogenous dispersion of the bacteria through the medium.

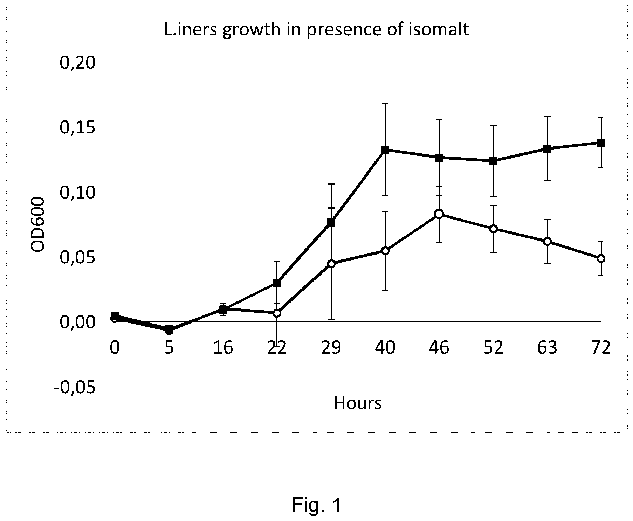

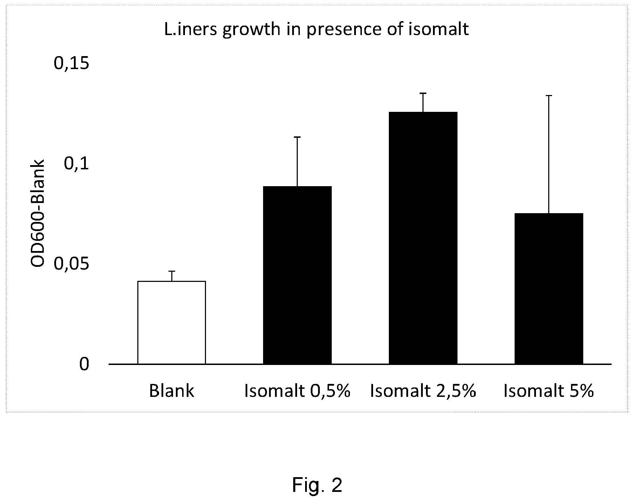

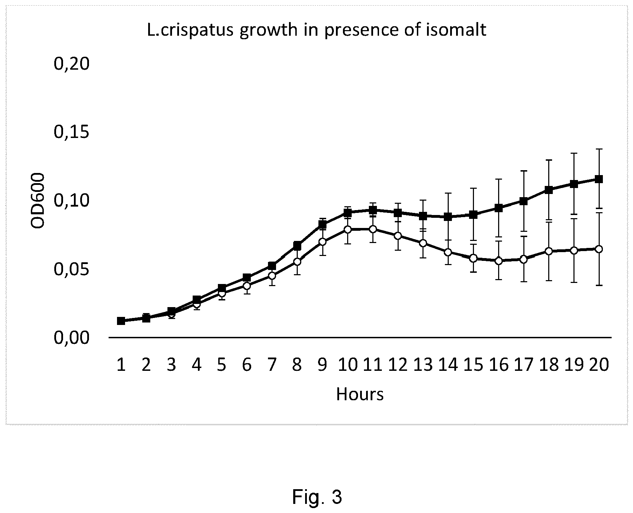

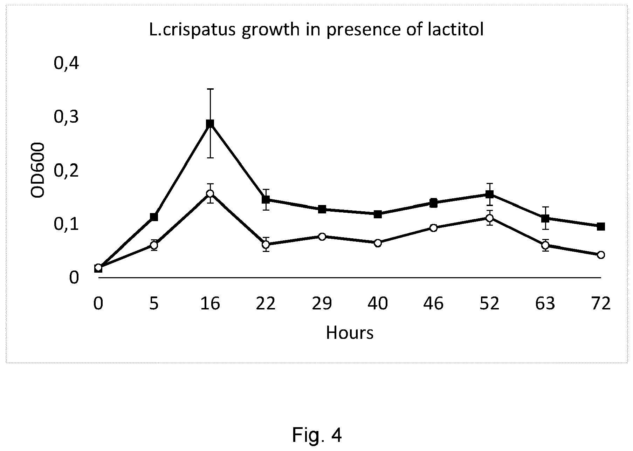

The results are presented in figures 1 to 4. As shown by bacterial growth data, isomalt promotes the growth of L. iners and L.crispatus.

## Claims

1. Isomalt, or a pharmaceutically acceptable salt thereof, for use in the treatment and/or prevention of a microbial infection in the urogenital system in a subject.

2. Isomalt, or a pharmaceutically acceptable salt thereof, for use according to claim 1, wherein the microbial infection is a bacterial infection.

3. Isomalt, or a pharmaceutically acceptable salt thereof, for use according to any one of claims 1 or 2, wherein the microbial infection in the urogenital system is bacterial vaginosis.

4. Isomalt, or a pharmaceutically acceptable salt thereof, for use according to any one of the preceding claims, wherein the microbial infection is a fungal infection, such as a candida infection.

5. Isomalt, or a pharmaceutically acceptable salt thereof, for use according to any one of the preceding claims, wherein the microbial infection in the urogenital system is a disease of the female genital system.

6. Isomalt, or a pharmaceutically acceptable salt thereof, for use according to claim 5, wherein the disease of the female genital system is female infertility, such as female infertility due to implantation failure or female infertility due to spontaneous abortions.

7. Isomalt, or a pharmaceutically acceptable salt thereof, for use according to claim 5, wherein the disease of the female genital system is cervicitis, such as chronic cervicitis, or endometritis, such as chronic endometritis.

8. Isomalt, or a pharmaceutically acceptable salt thereof, for use according to claim 1, wherein the microbial infection in the urogenital system is selected from the group consisting of urinary tract infection, urinary urge incontinence and cystitis, such as chronic cystitis.

9. Isomalt for use according to any one of the preceding claims, wherein the subject is a pregnant female.

10. Isomalt, or a pharmaceutically acceptable salt thereof, for use in the prevention of late miscarriage or preterm birth.

11. Isomalt, or a pharmaceutically acceptable salt thereof, for use according to claim 10, wherein the miscarriage or preterm birth is caused by a microbial infection.

12. Isomalt, or a pharmaceutically acceptable salt thereof, for use according to any one of the preceding claims, wherein the compound is in a pharmaceutical composition comprising:
a. 10 to 50 wt% isomalt, or a pharmaceutically acceptable salt thereof; and
b. 5 to 40 wt% Hypromellose (HPMC).

13. Isomalt, or a pharmaceutically acceptable salt thereof, for use according to claim 12, wherein the composition comprises or consists of: 10 to 50 wt% isomalt; 10 to 40 wt% HPMC; 10 to 25 wt% starch; 0.25 to 1.25 wt% silica; and 0.5 to 1.5 wt% magnesium stearate, with the proviso that the sum of the components does not exceed 100 wt%.

14. A pharmaceutical composition comprising:
a. 10 to 50 wt% isomalt or a pharmaceutically acceptable salt thereof; and
b. 5 to 40 wt% Hypromellose (HPMC).

15. The pharmaceutical composition according to claim 14, wherein the pharmaceutical composition comprises or consists essentially of about: 30 to 35 wt% isomalt; 10 to 20 wt% HPMC; 30 to 40 wt% MCC; 12 to 18 wt% starch; 0.25 to 1.25 wt% silica; and 0.5 to 1.5 wt% magnesium stearate, with the proviso that the sum of the components does not exceed 100 wt%.
